(19) Europäisches Patentamt
European Patent Office
Office européen des brevets

(11) Veröffentlichungsnummer: **0 185 623**
**A1**

(12) # EUROPÄISCHE PATENTANMELDUNG

(21) Anmeldenummer: **85810603.2**

(22) Anmeldetag: **16.12.85**

(51) Int. Cl.⁴: **C 07 H 17/08**
A 61 K 31/70, A 01 N 43/24

(30) Priorität: **21.12.84 CH 6103/84**

(43) Veröffentlichungstag der Anmeldung:
**25.06.86 Patentblatt 86/26**

(84) Benannte Vertragsstaaten:
**AT BE CH DE FR GB IT LI LU NL SE**

(71) Anmelder: **CIBA-GEIGY AG**
**Klybeckstrasse 141**
**CH-4002 Basel(CH)**

(72) Erfinder: **Gehret, Jean-Claude, Dr.**
**Im Aeschfeld 12**
**CH-4147 Aesch(CH)**

(72) Erfinder: **Sturm, Elmar, Dr.**
**Klusstrasse 66**
**CH-4147 Aesch(CH)**

(72) Erfinder: **Frei, Bruno, Dr.**
**Bündtenstrasse 16**
**CH-4410 Liestal(CH)**

(54) **Milbemycin-Derivate zur Bekämpfung von Insekten und Parasiten.**

(57) Milbemycin D-Derivative der Formel 1 mit einem Kohlenhydratrest in der 5-Position

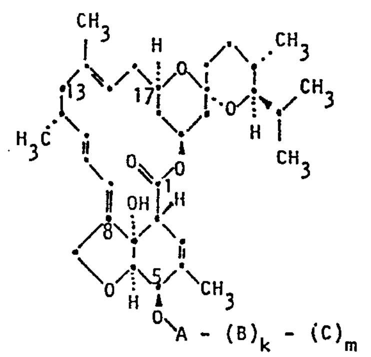

(I)

worin A, B, C, k und m die hierin gegebenen Definitionen besitzen, lassen sich zur Bekämpfung von Insekten, Ekto- und Endoparasiten einsetzen, insbesondere gegen tierparasitäre Nematoden. Die Verbindungen lassen sich als solche oder in Form geeigneter Mittel verwenden.

EP 0 185 623 A1

Croydon Printing Company Ltd

BEZEICHNUNG GEÄNDERT
siehe Titelseite



0185623

CIBA-GEIGY AG
Basel (Schweiz)

5-15208/+

# Mittel zur Bekämpfung von Insekten und Parasiten

Die vorliegende Erfindung betrifft Milbemycin D-Derivate der Formel I

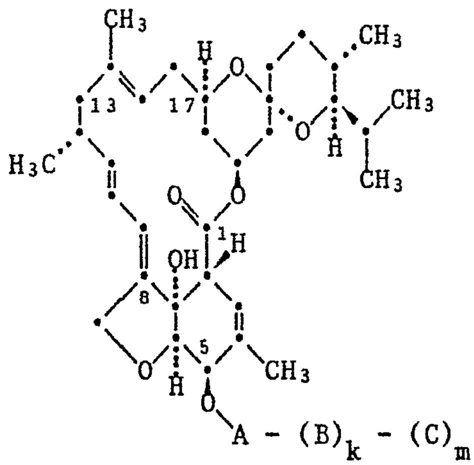

(I)

worin in 5-Position ein in 1'-Stellung verknüpfter Kohlenhydratrest A steht, der in 2'-Stellung eine leicht abspaltbare, über Sauerstoff gebundene Gruppe oder eine Hydroxigruppe besitzt, und der glykosidisch an ein zweites oder drittes Kohlehydratmolekül B und/oder C beliebiger Struktur geknüpft sein kann, wobei k und m unabhängig voneinander 0 oder 1 bedeuten.

Die Erfindung betrifft ferner die Herstellung der Verbindungen der Formel I und ihre Verwendung zur Bekämpfung von Schädlingen wie Ekto- und Endoparasiten, sowie Schädlingsbekämpfungsmittel, die mindestens eine dieser Verbindungen als Wirkstoff enthalten.

Formel I repräsentiert demgemäss Milbemycin D-Abkömmlinge, die in 5-Position ein Kohlehydrat, insbesondere ein Mono-, Di- oder Trisaccharid besitzen, das neben der Verknüpfungsstelle zum Makrolid eine OH-Gruppe trägt, die vorzugsweise derivatisiert ist.

Als Zuckerreste, die in 2'-Stellung wie angegeben substituiert sind, kommen somit in der Furanosylform bzw. in der Pyranosylform vorwiegend z.B. folgende Reste in Frage:

Monosaccharide: Glucose, Fructose, Altrose, Mannose, Sorbose, Gulose, Idose, Allose, Galactose, Ribose, Arabinose, Xylose, Lyxose, Erythrose, Threose, Rhamnose, Talose sowie ihre entsprechenden Derivate wie Methylglukose, Trimethylglukose und Tetraacetylglukose sowie ein- oder mehrfach acetylierte Zucker.

Disaccharide: Lactose, Maltose, Cellobiose, Melibiose, Gentiobiose sowie ihre entsprechenden Derivate.

Weiterhin rechnen zu den für Formel I genannten Kohlehydraten Saccharide, die zusätzlich einen Aminorest, einen Thiolrest oder einen aus zwei nachbarständigen OH-Gruppen und einem Aldehyd bzw. Keton gebildeten cyclischen Acetalrest enthalten.

Die Verknüpfung eines Saccharids in 5-Position des Milbemycin D kann als α-Anomeres oder β-Anomeres erfolgen. Die vorliegende Erfindung bezieht sich auf beide Bindungsarten.

Als leicht abspaltbare über Sauerstoff gebundene Gruppe in der 2'-Stellung des Zuckerrestes sind Methyl, Benzyl, eine unsubstituierte oder halogensubstituierte $C_1$-$C_6$-aliphatische Acylgruppe, eine Benzoylgruppe oder eine $C_1$-$C_6$-Alkoxicarbonylgruppe zu nennen.

Im Rahmen der vorliegenden Erfindung bedeutet Halogen vor allem Fluor, Chlor oder Brom.

Zur Bildung eines cyclischen Acetals an einem Zuckermolekül eignen sich einfache Aldehyde wie Acetaldehyd, Propionaldehyd, Butyraldehyd, Benzaldehyd oder Ketone wie Acetophenon, Cyclopentanon, Cyclohexanon, Cycloheptanon, Fluorenon, Methylethylketon, vor allem aber Aceton unter Bildung entsprechender Acetonide.

Die Erfindung bezieht sich im engeren Sinne auf unter die Formel I fallende Milbemycin D-Derivate der Formel Ia mit Einschluss aller stellungsisomeren Reste im Zuckerteil:

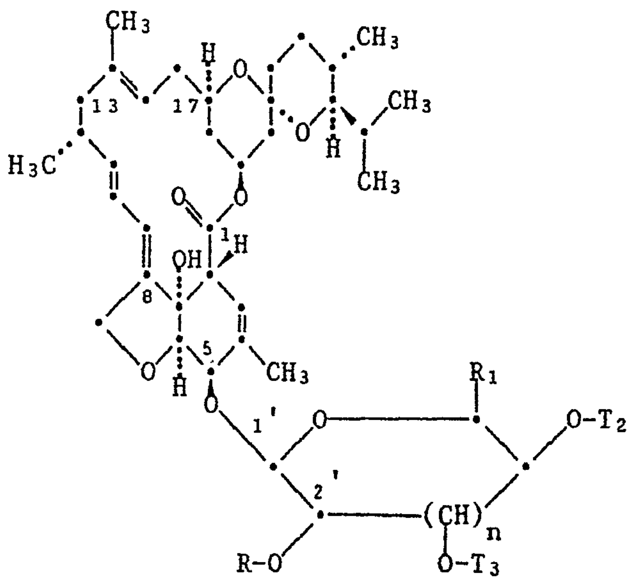

(Ia)

worin n einen Zahlenwert 0 oder 1 darstellt, $R_1$ Wasserstoff, Methyl oder $-CH_2-O-T_1$ bedeutet und R, $T_1$, $T_2$ und $T_3$ unabhängig voneinander Wasserstoff, Methyl, Benzyl, eine unsubstituierte oder halogensubstituierte $C_1$-$C_6$-aliphatische Acylgruppe, eine Benzoylgruppe, oder eine $C_1$-$C_6$-Alkoxicarbonylgruppe bedeuten, oder wobei $T_1$ und $T_2$ zusammen mit dem Carbonyl-Kohlenstoffatom eines aliphatischen oder aromatischen Aldehyds oder Ketons mit maximal 13 Kohlenstoffatomen ein cyclisches Acetal bilden.

Unter den vorgenannten Milbemycin D-Derivaten der Formel I bzw. Ia sind solche bevorzugt, worin die in 2'-Stellung befindliche über Sauerstoff gebundene Gruppe R Methyl, Benzyl, Benzoyl, unsubstituiertes oder durch Fluor substituiertes Propionyl oder Acetyl,

Methoxicarbonyl oder Ethoxicarbonyl bedeutet. Besonders bevorzugt sind unter diesen jene, worin diese Gruppe R Propionyl oder Acetyl ist.

Bevorzugt sind auch jene Milbemycin D-Derivate der Formel Ia, worin n 0 oder 1 ist, und $T_1$, $T_2$ und $T_3$ unabhängig voneinander Methyl, Benzyl, Benzoyl oder unsubstituiertes oder durch Fluor substituiertes Propionyl oder Acetyl bedeuten.

Bevorzugt sind ferner jene Milbemycin D-Derivate der Formel Ia, worin $T_1$ und $T_2$ zusammen mit dem Carbonyl-Kohlenstoffatom des Acetons oder des Benzaldehyds ein Acetonid bzw. ein Benzaldehyd-Acetal bilden.

Hervorzuheben sind ferner Verbindungen der Formel Ia, in welchen R, $T_1$, $T_2$ und, sofern n für 1 steht, auch $T_3$ Acetyl bedeuten, sowie weiterhin diejenigen Verbindungen der Formel Ia, in denen R, $T_1$, $T_2$ und, sofern n für 1 steht, auch $T_3$ Propionyl bedeuten.

Unter geschützten OH-Gruppen sind vorzugsweise solche zu verstehen, in denen das Wasserstoffatom durch $T_1$, $T_2$ oder $T_3$ ersetzt ist, wobei $T_1$, $T_2$ und $T_3$ die vorstehend angegebenen Bedeutungen haben.

In der US-Patentschrift Nr. 4 134 973 werden anders strukturierte Milbemycin-Derivate mit beliebigen Zuckerresten am Molekül vorgeschlagen. Ueberraschend kann jedoch mit vorliegender Erfindung gezeigt werden, dass Milbemycin D-Derivate mit der vorliegenden speziellen Struktur der Formel I eine sprunghaft verbesserte biologische Wirkung, insbesondere gegen Endo-Parasiten, im Vergleich zu der strukturnächsten Verbindung der genannten Patentschrift, nämlich zu 5-O-(2,3,4,6-Tetra-O-acetyl-β-O-glycopyranosyl)-milbemycin $A_3$, entfalten. Hinweise zu einer derartig weitreichenden Wirkungsverbesserung können der Lehre der US-PS 4 134 973 in keiner Weise entnommen werden. Es wird dort im

Gegenteil sogar gesagt, dass der Zuckerrest an verschiedenen Stellen des Moleküls gebunden sein kann und dass die Art des Zuckerrestes keine kritische Grösse darstelle.

Die Herstellung von Verbindungen der Formel I stellt eine Derivatisierung der sehr reaktionsfähigen 5-Hydroxigruppe im Milbemycin D mit einem geeigneten Kohlehydratmolekül dar und wird nach einer der in der Zuckerchemie verwendeten Verknüpfungsmethoden, z.B. nach der Koenigs-Knorr-Methode, dem Ag-Triflat-Prozess, nach dem sogenannten Orthoester-Verfahren, der Phenylthio-Synthese oder der 2-Pyridylthio-Methode durchgeführt.

A) Nach der Koenigs-Knorr-Methode oder dem Ag-Triflat-Prozess lässt sich Milbemycin D der Formel II

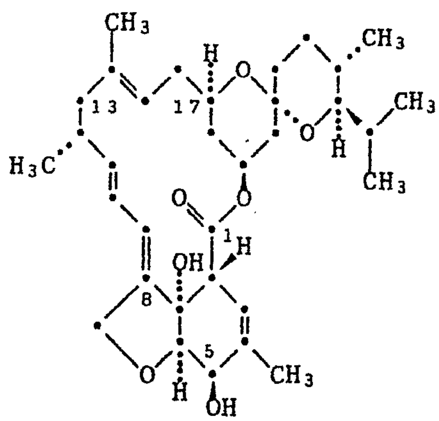

(II)

in Gegenwart eines Silbersalzes oder Quecksilber-Salzes als Kondensationsmittel mit dem einzuführenden Kohlehydrat A oder $A-(B)_k-(C)_m$, worin A, B, C, k und m die für Formel I gegebene Bedeutung haben, die anomere, in 1-Position befindliche OH-Gruppe durch ein Chlor- oder Bromatom ersetzt ist und alle anderen OH-Gruppen geschützt sind, im Temperaturbereich von -30°C bis +60°C, bevorzugt -5°C bis +30°C, unter Licht-Ausschluss umsetzen. Das gewünschte Kohlehydrat kann, sofern in 5-Position der Formel II ein Rest $A-(B)_k-(C)_m$, in welchem mindestens eines der Symbole k und m für 1 steht, addiert

werden soll, schrittweise an Milbemycin D gebunden werden, oder es kann vorzugsweise als bereits fertig vorgebildetes Glykosid in einem einzigen Reaktionsschritt mit Milbemycin D verknüpft werden.

Als Silbersalz lässt sich frisch gefälltes $Ag_2O$ verwenden, vorzugsweise jedoch $Ag_2CO_3$ oder $CF_3$-COOAg. Besonders bevorzugt ist Silber-Trifluormethansulfonat (Ag-Triflat = $CF_3$-$SO_3$Ag), in dessen Gegenwart die Glykosidierung bereits bei Minustemperaturen schnell abläuft. Zum Aktivieren der 5-OH-Gruppe des Milbemycin D und zum Neutralisieren von gegebenenfalls entstehendem $CF_3$-$SO_3$H bzw. $CF_3$-COOH setzt man zweckmässig ein tert.Amin (beispielsweise Triethylamin, Diisopropylethylamin oder Diazabicycloundecan) der Reaktionslösung zu.

Sofern gewünscht, können die Schutzgruppen durch milde Verseifung (z.B. mit $NH_3/CH_3OH$) anschliessend abgespalten werden.

Als Lösungsmittel kommen wasserfreie aprotische Vertreter wie Dichlormethan, Acetonitril, Benzol, Toluol, Nitromethan, Dioxan, Tetrahydrofuran oder Ethylenglykoldimethylether in Frage; Diethylether ist besonders geeignet.

Das geschützte 1-Chlor- oder 1-Brom-Kohlehydrat wird, bezogen auf Milbemycin D (Formel II), in äquimolarer Menge eingesetzt, vorzugsweise jedoch in einem 1,5- bis 3-fachen Ueberschuss. Die Reaktionsdauer beträgt, um eine befriedigende Ausbeute zu erzielen, 5 bis 72 Stunden.

Anstelle des Silbersalzes lässt sich auch Hg-Cyanid oder eine Kombination von HgO mit wahlweise Hg-Chlorid oder Hg-Bromid verwenden (Helferich-Synthese).

Nach einer weiteren Variante lässt sich die Reaktivität in der 1-Stellung des glykosidisch zu verknüpfenden Kohlehydrats, dessen weitere OH-Gruppen geschützt sein müssen, dadurch erhöhen, dass man die anomere, in 1-Position befindliche OH-Gruppe durch ein Fluoratom ersetzt, was zweckmässigerweise durch anfängliche Umwandlung des

entsprechenden Kohlehydrats in das 1-Phenylthio-Derivat und anschliessende Reaktion mit DAST (= Diethylaminoschwefeltrifluorid) in absolut trockenem Dichlormethan (Molekularsieb) bei +5°C bis -30°C erfolgt. Reaktiver als das entsprechende, bei der Koenigs-Knorr-Synthese eingesetzte 1-Chlor- oder 1-Brom-Derivat, lässt sich das so gewonnene 1-Fluor-Derivat des Kohlehydrat-Reaktanden mit Milbemycin D (Formel II) in Gegenwart von $SnCl_2$ und $AgClO_4$ in einem trockenen aprotischen Lösungsmittel wie Diethylether unter Schutzgas wie Argon bei +5°C bis -30°C verknüpfen. (J. Am. Chem. Soc. 1984, 106, 4189-4192).

B) Eine bessere Reaktion des glykosidisch zu verknüpfenden Kohlehydrats mit Milbemycin D der Formel II wird erzielt, wenn das in 1-Stellung zu aktivierende Kohlehydrat, dessen OH-Gruppen geschützt sind, mit 2,2'-Dithiodiopyridin in trockenem Dichlormethan bei ca. 0°C und unter Argon-Atmosphäre in das 1-(2-Pyridylthio)-Kohlehydrat überführt wird, das mit der freien 5-OH-Gruppe des Milbemycin D in Gegenwart von $Pb(ClO_4)_2$ als Kondensationsmittel bei Raumtemperatur in Tetrahydrofuran (THF) als Lösungsmittel leicht unter Bildung der glykosidischen Bindung reagiert. (J. Org. Chem. 1983, 48, 3489-3493).

C) Glykosidische Verknüpfungen lassen sich auch in Gegenwart von Lewis-Säuren erzielen, wie $AlCl_3$, $AlBr_3$, $SnCl_4$, $ZnCl_2$, $BF_3$, insbesondere als $(C_2H_5)_2O(BF_3)$ (Bortrifluorid-ätherat), wozu insbesondere acetylierte Zucker sehr geeignet sind. (Chimia 21, 1967, S. 537-538).

D) Nach der sogenannten Orthoester-Methode lassen sich glykosidische Bindungen auch durch Reaktion von Milbemycin D mit dem zu verknüpfenden OH-geschützten Zucker in Gegenwart des Orthoesters eines niederen Alkohols erzielen, dessen eine alkoholische Komponente der Zucker-Reaktand ist.

Ein weiterer Gegenstand vorliegender Erfindung betrifft Schädlingsbekämpfungsmittel, welche gegen Ekto- und Endoparasiten sowie Schadinsekten wirksam sind und neben üblichen Trägerstoffen und/oder Verteilungsmitteln als mindestens einen Wirkstoff eine Verbindung der Formel I enthalten.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen der Formel I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen der Formel I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizide gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rhizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie

Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen aüf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10 000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln mindestens eine Verbindung der Formel I als Wirkstoff enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiele

H-1. Herstellung von 5-O-(2,3,4,6-Tetra-O-acetyl-1-O-galactopyranosyl)-milbemycin D

1,12 g (2 mmol) Milbemycyn D und 1,65 g (4 mmol) Acetobromgalactose werden bei Raumtemperatur in ca. 70 ml trockenem Diethylether gelöst. Unter kräftigem Rühren werden zuerst 0,76 g (6 mmol) Diisopropylethylamin und anschliessend 0,77 g (3 mmol) Silber-Triflat zugegeben. Unter Lichtausschluss wird 20 Std. bei Raumtemperatur gerührt. Nach dem Filtrieren wird eingeengt und der verbleibende Rückstand über eine 20 cm langen Kolonne von Silicagel gereinigt (Laufmittel Dichlormethan/Diethylether, 3:2). Man erhält das gewünschte Produkt als amorphes Pulver; Smp. 130-135°C.

H-2. Herstellung von 5-O-(1-O-Galactopyranosyl)-milbemycin D

890 mg (1 mmol) des unter H-1 erhaltenen Tetraacetylgalactosids werden über Nacht in 25 ml einer methanolischen Lösung mit 25 % $NH_3$ bei 4-8°C gerührt. Nach dem Einengen wird das erhaltene Rohprodukt über eine Silicagel-Kolonne (Laufmittel Dichlormethan/Methanol 9:1) gereinigt; Smp. 120-125°C.

H-3. Herstellung von 5-O-(2,3,4,6-Tetra-O-acetyl-1-O-glucopyranosyl)-milbemycin D

0,56 g (1 mmol) Milbemycin D und 0,83 g (2 mmol) Acetobromglucose werden bei Raumtemperatur in 40 ml Dichlormethan gelöst. Unter kräftigem Rühren werden zuerst 0,38 g (3 mmol) Diisopropylethylamin und anschliessend 0,55 g (2 mmol) frisch bereitetes $Ag_2CO_3$ (oder $Ag_2O$) zugegeben. Unter Lichtausschluss wird 24 Std. bei Raumtemperatur gerührt. Nach dem Filtrieren wird eingeengt und über eine Silicagel-Kolonne gereinigt (Ethylacetat/Cyclohexan 4:1). Man erhält das Endprodukt als weisses Pulver; Smp. 120-126°C.

H-4. Herstellung von 5-O-(2,3,4,6-Tetra-O-acetyl-1-O-glucopyranosyl)-milbemycin D

Eine Lösung von 0,4 g (0,72 mmol) Milbemycin D in 16 ml abs. Tetrahydrofuran (THF) wird unter Argonatmosphäre durch eine Kanüle zu 0,32 g (0,72 mmol) S-Pyridyl-tetraacetylglucose gegeben und dann bei -30°C mit einer Lösung von 1,7 g (7,83 mmol) $AgClO_4$ in 15 ml abs. THF versetzt, wobei sofort ein weisser Niederschlag gebildet wird. Das Gemisch wird 30 Min. bei -30°C gerührt und dann unter Rühren mit 6 ml Triethylamin versetzt, auf Raumtemperatur erwärmt, mit 50 ml Ether verdünnt und rasch durch 10 g $SiO_2$ filtriert. Das Lösungsmittel wird am Rotationsverdampfer entfernt, und die Chromatographie ergibt das Produkt als weisses amorphes Pulver; Smp. 120-126°C.

H-5. Herstellung von 5-O-(2,3,4,6-Tetra-O-methyl-1-O-glucopyranosyl)-milbemycin D

600 mg (1,07 mmol) Milbemycin D und 500 mg (1,67 mmol) 1-Brom-2,3,4,6-tetramethylglucose werden in Argonatmosphäre in trockenem Diethylether gelöst. Danach werden 417 mg (3 mmol) Diisopropylethylamin zugegeben und das Reaktionsgemisch wird 5 Min. gerührt. Dann gibt man bei Raumtemperatur 514 mg (2 mmol) Ag-Triflat dazu, wobei die Temperatur von 22°C auf 27°C steigt und lässt unter

Lichtausschluss 15 Std. rühren. Der beige-farbene Nierderschlag wird abfiltriert, das Filtrat mit Diethylether verdünnt und zweimal mit je 10 ml 1 N $NaHCO_3$-Lösung und dann zweimal mit je 10 ml Wasser gewaschen. Nach dem Trocknen über $Na_2SO_4$ wird die Lösung eingeengt und über Silicagel (Laufmittel Dichlormethan/Methanol 19:1) gereinigt. Man erhält 480 mg des Endprodukts.

Massenspektrum m/e: 774 ($M^+$), 756, 555, 538, 428.

$^1H$-NMR (300 $MH^z$; $CDCl_3$; $Si(CH_3)_3$ = TMS);

3,30 (m) ($C_2H$, d.h. Signal des Protons in der 2-Position des Makrolids).

3,40 (s)
3,50 (s)
3,54 (s)
3,62 (s)  ] vier Acetyl-$CH_3$

4,0 (d) ($C_6H$)

4,96 (t) ($C_{15}H$)

Auf diese Art oder nach Art einer der weiter oben genannten Methoden lassen sich folgende Milbemycin D-Derivate der Formel I gewinnen (k = m = 0), die in der Regel in der Pyranosidform vorliegen.

| Verb. Nr. | A | Physikalische Daten |
|---|---|---|
| 1.1 | 2,3,4,6-Tetra-O-acetyl-glucose | Smp. 120-126°C |
| 1.2 | 6-O-Acetyl-glucose | Smp. 172-174°C |
| 1.3 | 2,3,4,6-Tetra-O-propionyl-glucose | Smp. 81-85°C |
| 1.4 | 2,3,4,6-Tetro-O-methoxicarbonyl-glucose | |
| 1.5 | 6-O-Benzoyl-glucose | |
| 1.6 | 2,3,4,6-Tetra-O-benzoyl-glucose | |
| 1.7 | 2,3,4,6-Tetra-O-trifluormethyl-glucose | |
| 1.8 | 2-O-Acetyl-glucose-4,6-acetonid | |
| 1.9 | 2-O-Acetyl-glucose-4,6-benzaldehydacetal | |
| 1.10 | 2,3,4,6-Tetra-O-methyl-glucose | (Beispiel H-5) |
| 1.11 | 2,3,4,6-Tetra-O-butyryl-glucose | |
| 1.12 | 2,3,4,6-Tetra-O-butyryl-galactose | |
| 1.13 | 2,3,4,6-Tetra-O-methoxicarbonyl-galactose | |
| 1.14 | 2,3,4,6-Tetra-O-benzoyl-galactose | |
| 1.15 | 2,3,4,6-Tetra-O-acetyl-galactose | Smp. 130-135°C |
| 1.16 | 6-O-Acetyl-galactose | Smp. 120-125°C |
| 1.17 | 2,3,4,6-Tetra-O-propionyl-galactose | |
| 1.18 | 2,3,4,6-Tetra-O-ethoxicarbonyl-galactose | |
| 1.19 | 2-O-Acetyl-galactose-4,6-acetonid | |
| 1.20 | 2,3-Di-O-acetyl-galactose-4,6-acetonid | |
| 1.21 | 2,3-Di-O-acetyl-galactose-4,6-benzaldehyd-acetal | |
| 1.22 | 2,3,5-Tri-O-acetyl-ribose (Furanosid) | Smp. 110-114°C |
| 1.23 | 5-O-Acetyl-ribose (Furanosid) | Smp. 149-153°C |
| 1.24 | 2,3,5-Tri-O-propionyl-ribose (Furanosid) | |
| 1.25 | 2,3,5-Tri-O-methoxicarbonyl-ribose (Furanosid) | |
| 1.26 | 2-O-Acetyl-ribose-3,5-acetonid (Furanosid) | |
| 1.27 | 2,3,4-Tri-O-acetyl-xylose | $m/e^+$ = 814 ($m^+$), 736, 598, 554, 1H-NMR: 2,10 (s) und 2,14 (s) 2 $COCH_3$, 3,3 (m) ($C_2H$), 4,96 (t) ($C_{15}H$) |

| Verb. Nr. | A | Physikalische Daten |
|---|---|---|
| 1.28 | 2,3,4-Tri-O-propionyl-xylose | |
| 1.29 | 2,3,4-Tri-O-methyl-xylose | |
| 1.30 | 2,3,4-Tri-O-benzoyl-xylose | |
| 1.31 | 2,3,4-Tri-O-acetyl-rhamnose | $m/e^+ = 828$ ($m^+$) 701, 598, 556, 538; 1H-NMR: 1,24 (d) ($C_5{}'CH_3$); 3,3 (m) ($C_2H$); 4,96 (t) ($C_{15}H$) |
| 1.32 | 2,3,4-Tri-O-methoxicarbonyl-rhamnose | |
| 1.33 | 2,3,4-Tri-O-propionyl-rhamnose | |
| 1.34 | 2,3,4-Tri-O-buturyl-rhamnose | |
| 1.35 | 2,3,4-Tri-O-acetyl-arabinose | |
| 1.36 | 2,3,4-Tri-O-propionyl-arabinose | |
| 1.37 | Glucose | Smp. 146-149°C |
| 1.38 | Galactose | Smp. 120-125°C |
| 1.39 | Ribose | Smp. 126-131°C |
| 1.40 | Xylose | |
| 1.41 | Rhamnose | |
| 1.42 | Arabinose | |
| 1.43 | 4-O-Acetyl-xylose | |

sowie auch folgende Milbemycin D-Derivate der Formel I mit 5-O-Disaccharid-Resten:

| Verb. Nr. | $A-(B)_k-(C)_m$ | Physikalische Daten |
|---|---|---|
| 2.1 | Cellobiose-hepta-acetat | |
| 2.2 | Lactose-hepta-acetat | |
| 2.3 | Cellobiose | |
| 2.4 | Lactose | |

Formulierungsbeispiele für den Wirkstoff der Formel I

(% = Gewichtsprozent)

| Spritzpulver | a) | b) | c) |
|---|---|---|---|
| Wirkstoff Nr. 1.1-1.43 oder 2.1-2.4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff Nr. 1.1-1.43 oder 2.1-2.4 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff Nr. 1.1-1.43 oder 2.1-2.4 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff Nr. 1.1-1.43 oder 2.1-2.4 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Tabletten bzw. Boli

| | | |
|---|---|---|
| I | Ein Wirkstoff Nr. 1.1-1.43 oder 2.1-2.4 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen, dann Kieselsäure unter Rühren hinzugeben und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen.

III Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirksubstanz vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. sie enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24°C und 60 % relativer Luftfeuchtigkeit durchgeführt. Auswertungen und Zwischenauswertungen auf moribunde Tiere, Wachstum, Larven und Frassschäden erfolgen nach 24 Stunden, 48 Stunden und 72 Stunden.

Die Verbindungen der Formel I, wie beispielsweise Nr. 1.1, 1.27 und 1.43, erzielten bereits bei einer Wirkstoffkonzentration von 3 ppm eine vollständige Abtötung nach 24 Stunden.

B-2. Wirkung gegen pflanzenschädigende Akariden
OP-sensible Tetranychus urticae
Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25°C.

Nach sieben Tagen wird unter dem Binokular auf den Prozentsatz mobiler Stadien (Adulte und Nymphen) und auf vorhandene Eier ausgewertet.

Die Verbindungen der Formel I erzielten bereits bei einer Wirkstoffkonzentration von 0,4 ppm vollständige Abtötung.

B-3. Wirkung gegen $L_1$-Larven von Lucilia sericata
1 ml einer wässrigen Suspension der zu prüfenden Aktivsubstanz wird so mit 3 ml eines speziellen Larvenzuchtmediums bei ca. 50°C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Die Verbindungen der Formel I erzielten mit 250 ppm, die Verbindungen Nr. 1.1, 1.3, 1.15, 1.22, 1.27, 1.35, 1.37, 1.38 und 1.43 bereits bei 100 ppm eine Wirkung von 100 %.

B-4. Akarizide Wirkung gegen Boophilus microplus (Biarra-Stamm
Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken

aufgeklebt werden können. Jeder Zecke wird mit einer Injektionsnadel 1 µl einer Flüssigkeit injiziert, die eine 1:1-Mischung von Polyethylenglykol und Aceton darstellt und in der als bestimmte Wirkstoffmenge 0,5 µg pro Zecke gelöst ist. Kontrolltiere erhalten eine wirkstofffreie Injektion. Nach der Behandlung werden die Tiere unter Normalbedingungen in einem Insektarium bei ca. 28°C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Die Verbindungen Nr 1.1, 1.15, 1.22, 1.27, 1.31, 1.37 und 1.38 erzielen eine $IR_{90}$ von 0,5 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 2 mg oder 1 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formel I bei 1 mg/kg behandelt wurden, zeigten im Vergleich zu unbehandelten, aber infizierten Vergleichsgruppen keinen Nematodenbefall (= komplette Reduktion der Wurmeier im Kot). Tetraacetylglucopyranosyl-milbemycin $A_3$ ist bei 2 mg/kg annähernd wirkungslos. Schafe, die mit 0,1 mg/kg Köpergewicht der Verbindung Nr. 1.1 (A) und der Verbindung Nr. 1.15 (B) behandelt wurden, zeigten im

Vergleich zur Kontrolle eine 77%ige (A) und eine 97%ige (B) Wirkung, während 5-O-(2,3,4,6-Tetra-O-acetyl-1-O-glucanopyranosyl)-milbemycin $A_3$ bei dieser Konzentration unwirksam war.

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird ermittelt, ob tote bzw. abgefallene Blattläuse einen Anteil von mindestens 80 % ausmachen. Nur bei dieser Wirkungshöhe wird ein Präparat als wirksam eingestuft.

Die Verbindungen der Formel I erzielten bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1%igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formel I, wie beispielsweise Nr. 1.15 und 1.43, bewirkten in diesem Test bei einer Konzentration von 1,6 ppm bereits nach einem Tag eine vollständige Abtötung sämtlicher Larven.

Patentansprüche

1. Milbemycin D-Derivate der Formel I

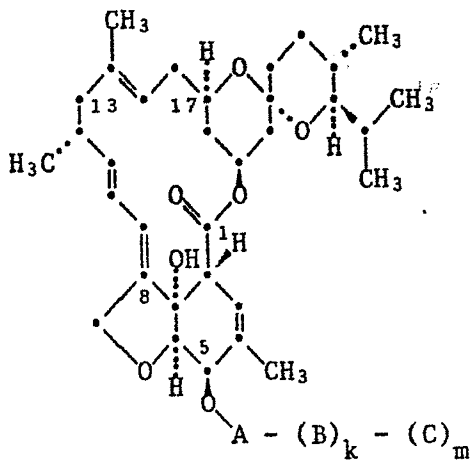

(I)

worin in 5-Position ein in 1'-Stellung verknüpfter Kohlenhydratrest A steht, der in 2'-Stellung eine leicht abspaltbare, über Sauerstoff gebundene Gruppe oder eine Hydroxigruppe besitzt, und der an ein zweites und/oder drittes Kohlehydratmolekül B und/oder C beliebiger Struktur geknüpft sein kann, wobei k und m unabhängig voneinander 0 oder 1 bedeuten.

2. Milbemycin D-Derivate der Formel Ia mit Einschluss der stellungs-isomeren Reste im Zuckerteil

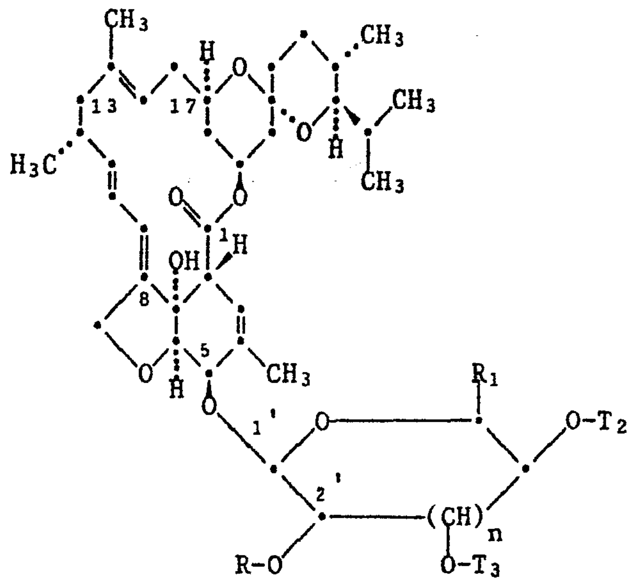

(Ia)

worin n einen Zahlenwert 0 oder 1 darstellt, und $R_1$ Wasserstoff, Methyl oder $-CH_2-O-T_1$ bedeutet, R, $T_1$, $T_2$ und $T_3$ unabhängig voneinander Wasserstoff, Methyl, Benzyl, eine $C_1$-$C_6$-aliphatische Acylgruppe,die auch halogeniert sein kann, eine Benzoylglruppe oder eine $C_1$-$C_6$-Alkoxicarbonylgruppe bedeuten, oder wobei $T_1$ und $T_2$ zusammen mit dem Carbonyl-Kohlenstoffatom eines aliphatischen oder aromatischen Aldehyds oder Ketons mit maximal 13 Kohlenstoffatomen ein cyclisches Acetal bilden.

3. Verbindungen der Formel I bzw. Ia gemäss Anspruch 1 oder 2, worin die in 2'-Stellung befindliche über Sauerstoff gebundene Gruppe R Methyl, Benzyl, Benzoyl, unsubstituiertes oder durch Fluor substituiertes Propionyl oder Acetyl, oder Methoxicarbonyl oder Ethoxicarbonyl bedeutet.

4. Verbindungen der Formel Ia gemäss Anspruch 2, worin R Propionyl oder Acetyl bedeutet.

5. Verbindungen der Formel Ia gemäss einem der Ansprüche 2 bis 4, worin n 0 oder 1 ist und $T_1$, $T_2$ und $T_3$ unabhängig voneinander Methyl, Benzyl, Benzoyl oder unsubstituiertes oder durch Fluor substituiertes Propionyl oder Acetyl bedeuten.

6. Verbindungen der Formel Ia gemäss Anspruch 2, worin $T_1$ und $T_2$ zusammen mit dem Carbonyl-Kohlenstoffatom des Acetons oder des Benzaldehyds ein Acetonid bzw. ein Benzaldehyd-Acetal bilden.

7. Verbindungen der Formel Ia gemäss Anspruch 2, worin R, $T_1$, $T_2$ und, sofern n = 1 ist, $T_3$ Acetyl bedeuten.

8. Verbindudngen der Formel Ia gemäss Anspruch 2, worin R, $T_1$, $T_2$ und, sofern n = 1 ist, $T_3$ Propionyl bedeuten.

9. Verfahren zur Herstellung von Milbemycin D-Derivaten der Formel I

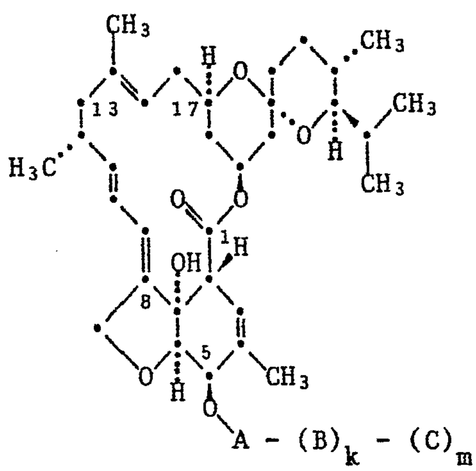

(I)

worin A, B, C, k und m die im Anspruch 1 gegebene Bedeutung haben, dadurch gekennzeichnet, dass man Milbemycin D
a) in Gegenwart eines Silbersalzes oder Quecksilber-Salzes als Kondensationsmittel mit dem einzuführenden Kohlenhydrat A oder $A-(B)_k-(C)_m$, worin A, B, C, k und m die für Formel I gegebene Bedeutung haben, die anomere, in 1-Position befindliche OH-Gruppe

durch ein Chlor- oder Bromatom ersetzt ist und alle anderen OH-Gruppen geschützt sind, unter Lichtausschluss im Temperaturbereich von -30°C bis +60°C, bevorzugt -5°C bis +30°C, oder
b) in Gegenwart von $SnCl_2$ und $AgClO_4$ als Kondensationsmittel mit dem einzuführenden Kohlenhydrat A oder $A-(B)_k-(C)_m$, worin A, B, C, k und m die für Formel I gegebene Bedeutung haben, die anomere, in 1-Position befindliche OH-Gruppe durch ein Fluoratom ersetzt ist und alle anderen OH-Gruppen geschützt sind, unter Lichtausschluss bei +5°C bis -30°C in einem aprotischen Lösungsmittel und unter Schutzgasatmosphäre umsetzt
und gewünschtenfalls durch milde Verseifung die Hydroxyl-Schutzgruppen abspaltet.

10. Schädlingsbekämpfungsmittel gegen Ekto- und Endoparasiten und Insekten, dadurch gekennzeichnet, dass es neben üblichen Trägerstoffen und/oder Verteilungsmitteln mindestens eine Verbindung der Formel I gemäss Anspruch 1 enthält.

11. Mittel gemäss Anspruch 10, dadurch gekennzeichnet, dass es als Verbindung der Formel I mindestens eine Verbindung gemäss einem der Ansprüche 2 bis 8 enthält.

12. Verwendung von Verbindungen der Formel I gemäss einem der Ansprüche 1 bis 8 zur Bekämpfung von Ekto- und Endoparasiten und Insekten an Tieren und Pflanzen.

13. Verwendung gemäss Anspruch 12 zur Bekämpfung von Endoparasiten im Warmblüter.

14. Verwendung gemäss Anspruch 13, dadurch gekennzeichnet, dass es sich bei den Endoparasiten um Nematoden handelt.

15. Verfahren zur Bekämpfung von Schädlingen an Tieren und Pflanzen, dadurch gekennzeichnet, dass man eine Verbindung der Formel I gemäss einem der Ansprüche 1 bis 8 auf oder in das Tier, auf die Pflanze oder deren Lebensraum appliziert.

FO 7.5/SES/ga*